# EUROPÄISCHE PATENTANMELDUNG

(11) **EP 2 898 919 A1**
(43) Veröffentlichungstag der Anmeldung: **29.07.2015**
(21) Anmeldenummer: 15150402.4
(22) Anmeldetag: 08.01.2015
(51) Int. Cl.: A61M 25/02

(54) **Einführvorrichtung**

(30) Priorität: 22.01.2014 US 201461929993 P
(71) Anmelder: Biotronik AG, 8180 Bülach (CH)
(72) Erfinder: Fargahi, Amir, 8180 Bülach (CH)
(74) Vertreter: Galander, Marcus

(57) **Zusammenfassung**

Die Erfindung betrifft eine Einführvorrichtung (100), insbesondere Führungskatheter (110) und/oder Ballonkatheter (120), zum Einführen in einen tierischen und/oder menschlichen Körper, wobei wenigstens ein Kathetersegment (10, 20) vorgesehen ist, bei dem ein Hohlraum (113, 32) und ein Fluidraum (115, 125) um einen zentralen Bereich (34) angeordnet sind, wobei der Hohlraum (113, 32) und der Fluidraum (115, 125) durch eine Wand (50) getrennt sind und die Wand (50) wenigstens eine Wandöffnung (52a, 52b) aufweist, die durch eine elastische Membran (40a, 40b) abgedichtet ist, und wobei die elastische Membran (40a, 40b) ausgebildet ist, bei einer ausreichenden Druckdifferenz zwischen Fluidraum (115, 125) und Hohlraum (113, 32) zur Anlage an einem der wenigstens einen Wandöffnung (52a, 52b) gegenüberliegend angeordneten Anlagebereich (111, 30) im Hohlraum (113, 32) zu gelangen.

## Beschreibung

Die Erfindung betrifft eine Einführvorrichtung, insbesondere einen Ballonkatheter oder eine Führungskatheter, zum Einführen in einen tierischen und/oder menschlichen Körper.

Es ist bekannt, zur perkutane transluminalen koronaren Angioplastie (PTCA) oder zur perkutane transluminalen Angioplastie (PTA) so genannte Ballonkatheter zu verwenden, um ein verengtes oder verschlossenes Blutgefäß zu erweitern, um einen ungestörten Blutfluss im Gefäß zu ermöglichen. Hierzu wird üblicherweise über ein Blutgefäß ein Führungskatheter eingeführt und der Ballonkatheter durch diesen zu der zu behandelnden Stelle vorgeschoben. Am distalen Ende des Ballonkatheters befindet sich ein Ballon, der in der Gefäßverengung expandiert wird, indem dieser mit einem Fluid mit Druck beaufschlagt wird. Weitere ähnliche Indikationen für den Einsatz von Ballonkathetern sind die so genannte Valvuloplasty und die so genannte Renal Sympathetic Denervation.

Der Ballon wird vor einem Einführen in den Körper mit Kathetern verbunden und muss so befestigt sein, dass dieser am Einsatzort komplikationslos genau platziert werden kann. Ein häufiges Problem ist hierbei, dass der Ballon verrutschen kann, was bei der Aufweitung des mit Druck beaufschlagten Ballons zu verschiedensten Komplikationen führen kann.

Aus der US 2010/0262076 A1 ist ein Ballonkatheter mit einem Führungsdraht und drei in Serie angeordneten Ballons bekannt. Ein Steuerballon dient zur Positionierung vor der Gefäßverengung bzw. dem Gefäßverschluss. Ein Stabilisierungsballon dient zur Fixierung des Ballonkatheters im Führungskatheter. Ein zwischen Steuerballon und Stabilisierungsballon angeordneter Ankerballon dient zur weiteren Verankerung im Körpergefäß.

Der Erfindung liegt die Aufgabe zugr- 2 -unde, eine verbesserte Einführvorrichtung anzugeben, mit der ein hochpräzises Platzieren der Einführvorrichtung, insbesondere eines damit verbundenen Ballons, erfolgen kann.

Die Aufgabe wird erfindungsgemäß durch die Merkmale des Anspruchs 1 gelöst. Günstige Ausgestaltungen und Vorteile der Erfindung ergeben sich aus den weiteren Ansprüchen, der Zeichnung und der Beschreibung.

Es wird eine Einführvorrichtung vorgeschlagen, insbesondere ein Führungskatheter und/oder Ballonkatheter, zum Einführen in einen tierischen und/oder menschlichen Körper, wobei wenigstens ein Kathetersegment vorgesehen ist, bei dem ein Hohlraum und ein Fluidraum um einen zentralen Bereich angeordnet sind, wobei der Hohlraum und der Fluidraum durch eine Wand getrennt sind und die Wand wenigstens eine Wandöffnung aufweist, die durch eine elastische Membran abgedichtet ist. Die elastische Membran ist dazu ausgebildet, bei einer ausreichenden Druckdifferenz zwischen Fluidraum und Hohlraum zur Anlage an einem der wenigstens einen Wandöffnung gegenüberliegend angeordneten Anlagebereich im Hohlraum zu gelangen.

Dabei kann je nach Ausgestaltung die Membran durch die wenigstens eine Wandöffnung durchgreifen oder von der Wandöffnung weggedrückt werden.

Beim Anlegen an den Anlagebereich sind Position und Orientierung von Kathetersegment und Anlagebereich zueinander fixiert. Wird der Druck wieder reduziert, kehrt die Membran in ihre Ausgangsposition zurück und Position und Orientierung wieder frei. Die Membran wirkt somit vereinfacht ausgedrückt als Bremse zur Fixierung. Vorteilhaft kann ein Fixierungssystem insbesondere für Ballonkatheter geschaffen werden, die unabhängig von der äußeren Ballonoberfläche ist. Diese kann wirkstoffbeladen sein und/oder zur Reduktion der Reibung hydrophil bzw. hydrophob beschichtet sein. Die wenigstens eine Wandöffnung kann als runde Öffnung oder als Langloch ausgestaltet sein.

Ein ausreichender Druck ist zweckmäßigerweise im Bereich des üblichen Nominaldrucks bei einer Ballonaufweitung, zumindest mindestens 50% des Nominaldrucks betragen. Mit Nominaldruck ist der Druck gemeint, mit dem der Ballon bei der jeweiligen Anwendung beaufschlagt wird. Die Elastizität der Membran kann entsprechend ausgelegt werden. Ein günstiger Druckbereich ist mindestens 0.5 bar, bevorzugt mindestens 1.0 bar, insbesondere 2-3 bar. In Abhängigkeit von der Anwendung kann auch der halbe Nominaldruck verwendet werden.

Gemäß einer günstigen Ausgestaltung kann der Anlagebereich um denselben zentralen Bereich wie Fluidraum und Hohlraum oder im zentralen Bereich angeordnet sein. Dies ergibt eine einfache und zuverlässige Geometrie, die das übliche Design von Kathetern nicht stört.

Gemäß einer günstigen Ausgestaltung kann die wenigstens eine Wandöffnung durch Perforierungen gebildet sein. Die durchbrochenen Bereiche eines Innen- oder Außenschlauchs behalten eine ausreichende Stabilität, während gleichzeitig durch eine Mehrzahl an Öffnungen, durch welche die Membran mit Druck beaufschlagt werden kann, eine ausreichende Haftkraft der Membran an ihrer Anlage aufgebracht werden kann.

Gemäß einer günstigen Ausgestaltung kann der Hohlraum den Fluidraum umgeben. Dies ist günstig in einem Führungskatheter. Die Membran kann beispielsweise an einer Außenseite eines Außenschlauchs angeordnet sein. In diesem Fall kann die Membran von eine Fluid aus dem innenliegenden Fluidraum, welches einen ausreichenden Druck aufbringt, von der wenigstens eine Wandöffnung weg nach außen in den Hohlraum und an eine Innenwand einer Hülle einer Schleuse oder eines Führungskatheters, je nach Indikation gedrückt werden.

Unter einer Schleuse wird ein einfacher Zugang (ähnlich einem Schlauch) in das jeweilige Körpergefäß verstanden. Dieser Zugang ist steril und im Körpergefäß im proximalen Bereich am Patienten fixiert. Derartige Schleusen werden vor allem bei peripheren Zugängen verwendet. Die Schleuse reicht nicht sehr weit in das Körpergefäß hinein.

Ein Führungskatheter wird bei komplexeren Anwendungen verwendet. Hier wird der eigentliche Katheter durch den Führungskatheter an die jeweilige Position der Anwendung geführt. Entsprechend reicht der Führungskatheter nahezu bis zum Anwendungsort in das Körpergefäß hinein. Der Führungskatheter ist ebenfalls am Ort des äußeren Zugangs in den Patienten im proximalen Bereich fixiert.

Damit ergeben sich für die erfindungsgemäße Bremse folgende Anwendungsfälle für die Fixierung des Katheters in Bezug auf den Patienten: der Katheter wird gegen die Schleuse fixiert oder der Katheter wird gegen den Führungskatheter fixiert.

Gemäß einer günstigen Ausgestaltung kann der Fluidraum den Hohlraum umgeben. Dies ist günstig in einem Ballonkatheter. Die Membran kann beispielsweise am Rand des Führungsdrahtlumens angeordnet sein. In diesem Fall kann die Membran von eine Fluid aus dem außenliegenden Fluidraum, welches einen ausreichenden Druck aufbringt, durch die wenigstens eine Wandöffnung in den Hohlraum durchgreifen und gegen den Führungsdraht der Einführvorrichtung gedrückt werden. Vorteilhaft kann dabei der Anlagebereich in einem Ballonkatheter angeordnet sein.

Gemäß einer günstigen Ausgestaltung kann der Ballon und/oder die Membran aus nachgiebigem Material wie z.B. Nylon oder Polyamid gebildet sein.

Gemäß einer weiteren günstigen Ausgestaltung kann der Ballon und/oder die Membran aus einem Material der Gruppe gebildet sein: Ethylen-Vinylacetat, Polyvinylchlorid, Olefinkopolymere, Olefinhomopolymere, Polyethylen, Polyethylen-Typen (wie PE-HD bzw. HDPE (mit schwach verzweigten Polymerketten, daher hohe Dichte, wobei "HD" für "high density" steht), PE-LD bzw. LDPE (mit stark verzweigten Polymerketten, daher geringe Dichte, wobei "LD" für "low density" steht), PE-LLD bzw. LLDPE (lineares Polyethylen niederer Dichte, dessen Polymermolekül nur kurze Verzweigungen aufweist, wobei "LLD" für "linear low density" steht), PE-HMW (hochmolekulares Polyethylen, wobei die Polymerketten länger sind als bei PE-HD, PE-LD oder PE-LLD, wobei "HMW" für "high molecular weight" steht), PE-UHMW (ultrahochmolekulares Polyethylen mit einer mittleren Molmasse von bis zu 6000 kg/mol und einer Dichte von 0,93-0,94 g/cm³, wobei UHMW" für "ultra high molecular weight" steht)), Polyetheretherketon (PEEK, als hochtemperaturbeständiger thermoplastischer Kunststoff, der zur Stoffgruppe der Polyaryletherketone gehört), Polyurethan, Silicone, Polyethylenterephthalat (PET, ein durch Polykondensation hergestellter thermoplastischer Kunststoff aus der Familie der Polyester), Polyetherblockamid (PEBA, ein thermoplastischer Elastomer, bekannt unter dem Handelsnamen PEBAX des Herstellers Arkema), Acrylonitril-Polymere, Acrylonitril-Kopolymere, Acrylonitrilmischungen, Harze, insbesondere ionomere Harze.

Gemäß einer günstigen Ausgestaltung kann bei Druckbeaufschlagung der elastischen Membran Führungsdraht, Ballonkatheter und Führungskatheter in ihrer relativen Orientierung zueinander fixierbar sein. Zwischen dem Ballon des Ballonkatheters, dem fixierten Führungsdraht und dem Führungskatheter findet in diesem Zustand keine relative Bewegung statt. Während der Ballon-Aufweitung drückt die Membran bzw. die Membranen den Führungsdraht und den Führungskatheter bzw. die Schleuse zusammen und hält diese fest. Damit wird die Position des Ballons fixiert. Nach dem Entspannen des Ballons kehrt die elastische Membran zu ihrem ursprünglichen Zustand zurück.

Denkbar ist auch, nur einen Teil der Komponenten zu fixieren, etwa nur den Ballon gegenüber dem Führungsdraht oder den Ballon gegenüber dem Führungskatheter. Hierzu kann die Fluidzuführung in der Einführvorrichtung entsprechend ausgestaltet sein.

Die Einführvorrichtung ist für einen Anwender einfach zu bedienen und erfordert keine zusätzliche Schulung und kann mit nur geringem Aufwand für PTA- und PTCA-Ballone realisiert werden.

Die Erfindung ist nachfolgend beispielhaft, anhand von in Zeichnungen dargestellten Ausführungsbeispielen, näher erläutert. Es zeigen in schematischer Darstellung:
- Fig. 1: eine Ansicht einer Einführvorrichtung mit Führungskatheter und Ballonkatheter;
- Fig. 2: einen Schnitt durch einen Bereich eines Ballonkatheters nach einem Ausführungsbeispiel der Erfindung mit entspanntem Ballon;
- Fig. 3: einen Schnitt durch einen Bereich des Ballonkatheters nach Figur 2 mit druckbeaufschlagtem und auf einem Führungsdraht fixierten Ballon;
- Fig. 4: einen Schnitt durch einen Bereich eines Führungskatheters nach einem Ausführungsbeispiel der Erfindung mit entspanntem Außenschlauch;
- Fig. 5: einen Schnitt durch einen Bereich des Führungskatheters nach Figur 4 mit druckbeaufschlagtem und an einer Wand fixiertem Außenschlauch;
- Fig. 6: einen Querschnitt durch Führungskatheter, Ballonkatheter und Führungsdraht Schnitt mit entspanntem Ballon; und
- Fig. 7: einen Querschnitt durch Führungskatheter, Ballonkatheter und Führungsdraht Schnitt mit druckbeaufschlagtem Ballon.

In den Figuren sind funktionell gleiche oder gleich wirkende Elemente jeweils mit denselben Bezugszeichen beziffert. Die Figuren sind schematische Darstellungen der Erfindung. Sie bilden nicht spezifische Parameter der Erfindung ab. Weiterhin geben die Figuren lediglich typischen Ausgestaltungen der Erfindung wieder und sollen die Erfindung nicht auf die dargestellten Ausgestaltungen beschränken.

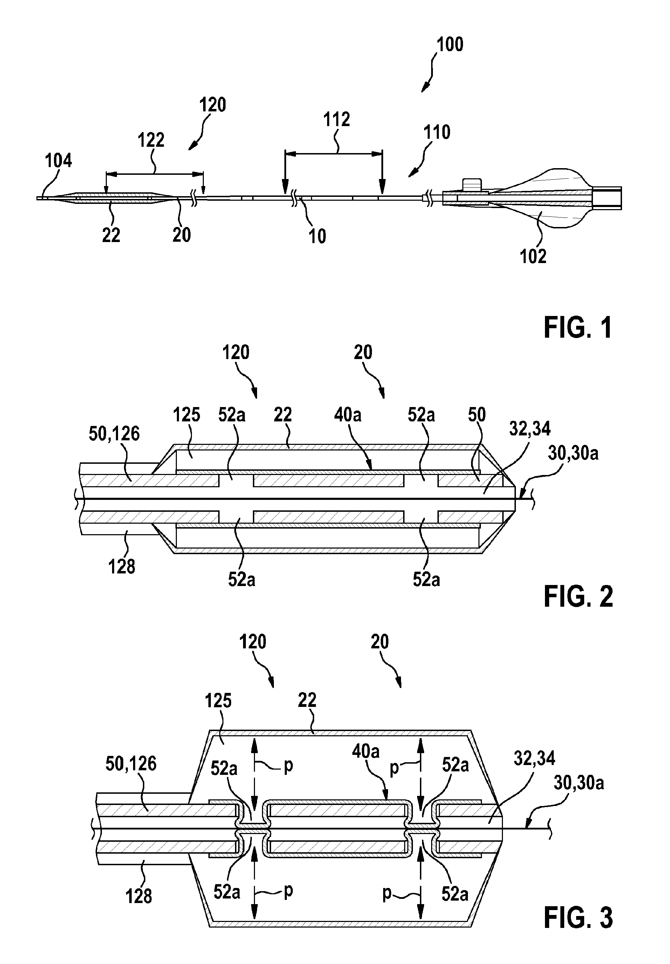

Figur 1 zeigt eine Ansicht einer Einführvorrichtung 100 mit einem Führungskatheter 110 an ihrem proximalen Ende 102 und einem Ballonkatheter 120 an ihrem distalen Ende 104. Durch den Ballonkatheter 120 ist ein Führungsdraht (nicht gezeichnet) geführt. Am distalen Ende 104 ist ein Ballon 20 angeordnet, der im Einsatzgebiet aufgeweitet werden soll, um eine Gefäßverengung oder einen Gefäßverschluss zu beseitigen.

Die Positionen 112 und 122 bezeichnen günstige Bereiche, um eine Fixierung des Führungskatheters 110 im Bereich 112 und des Ballonkatheters 120 im Bereich 122 vorzusehen. Es kann eine Fixierung in beiden Bereichen 112, 122 vorgesehen sein oder alternativ in nur einem der Bereiche 112, 122.

Figur 2 zeigt einen Schnitt durch ein Kathetersegment 20 eines Ballonkatheters 120 nach einem Ausführungsbeispiel der Erfindung mit entspanntem, nicht druckbeaufschlagtem Ballon 22. Durch den Ballonkatheter 120 ist in einem zentralen Bereich 34 ein Führungsdraht 30a in einem Hohlraum 32geführt. Der Ballonkatheter 120 weist zumindest einen Schlauch 126 (auch Schaft genannt) auf, der vom Ballon 22 umgeben ist und dessen Innenraum den zentralen Bereich 34 bildet. Zwischen Ballon 22 und der Außenseite des Schlauchs 126 ist ein Fluidraum 125 angeordnet, der den Schlauch 126 umgibt und durch welchen in üblicher Weise ein Fluid geleitet werden kann, um den Ballon 22 mit Druck zu beaufschlagen, wenn dieser aufgeweitet werden soll. Die Schlauchwand des Schlauchs 126 bildet eine Wand 50 zwischen Hohlraum 32 und Fluidraum 125.Die Ballonhülle geht am proximalen Ende in einen Außenschlauch 128 über, welcher den Schlauch 126 umgibt.

In dem Kathetersegment 20, in dem der Ballon 22 angeordnet ist, sind in der Wand 50, d.h. im Schlauch 126, Mantelöffnungen 52a vorgesehen. Die Mantelöffnungen 52a sind von einer elastischen Membran 40a abgedeckt. Im gezeigten Beispiel ist die Membran 40a im Kathetersegment 20 als Schlauchstück über den Schlauch 126 geschoben und umschließt den Schlauch 126 in diesem Bereich. Die Membran 40a befindet sich im Fluidraum 125. Denkbar ist auch eine unsymmetrische Anordnung, bei der die Membran 40a nur streifenweise oder einseitig am Umfang des Schlauchs 126 angeordnet ist. Fluidraum 125 und Hohlraum 32 umgeben den zentralen Bereich 34 und damit auch den Führungsdraht 30a.

Der Ballon 20 und/oder die Membran 40a kann durch biegsame Materialien gebildet sein, wie z.B. insbesondere Ethylen-Vinylacetat, Polyvinylchlorid, Olefinkopolymere, Olefinhomopolymere, Polyethylen-Typen (wie PE-HD bzw. HDPE, PE-LD bzw. LDPE, PE-LLD bzw. LLDPE, PE-HMW, PE-UHMW), Polyetheretherketon, Polyurethan, Silicone, Polyethylenterephthalat, Polyetherblockamid (PEBA), Acrylonitril-Polymere, Acrylonitril-Kopolymere, Acrylonitrilmischungen, Harze, insbesondere ionomere Harze. Andere geeignete Materialien können ebenfalls eingesetzt werden.

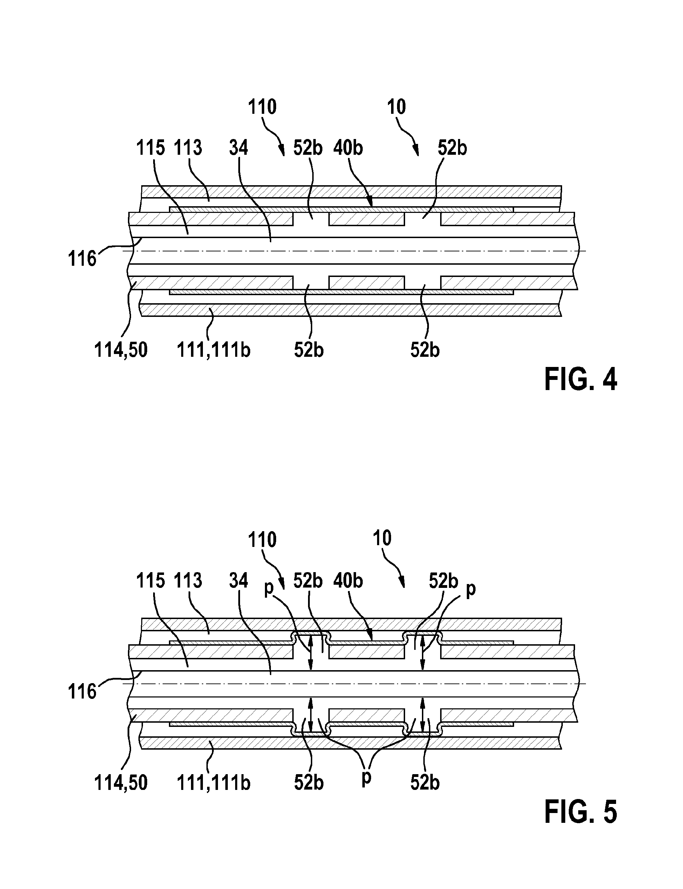

Figur 3 zeigt einen Schnitt durch das Kathetersegment 20 des Ausführungsbeispiels in Figur 2 mit druckbeaufschlagtem und aufgeweitetem Ballon 22. Durch das dem Fluidraum 125 zugeführte Fluid wird ein hoher Druck p von einigen Bar bis zu 20 bar aufgebaut und der Ballon 22 entsprechend aufgeweitet. Gleichzeitig drückt der vom Fluid aufgebaute Druck p die Membran 40a durch die Mantelöffnungen 52a nach innen in den zentralen Bereich 34 bis die Membran 40a an den Führungsdraht 30a stößt und diesen festklemmt. Der Führungsdraht 30a bildet in diesem Bereich einen Anlagebereich 30. Die Lage und die Orientierung von Ballon 22 und Führungsdraht 30a sind nunmehr festgelegt, solange die Membran 40a auf den Führungsdraht 30a drückt. Wird der Druck p abgebaut, bewegt sich die Membran 40a wieder von ihrem Anlagebereich 30 in ihre Ausgangsposition zurück und gibt den Führungsdraht 30a frei. Durch die quasi "konzentrische" Anordnung von Ballon 22, Fluidraum 125, Membran 40a, Schlauch 126, Hohlraum 32 und Führungsdraht 30a ist die Funktion der Fixierung des Ballons 22 gegenüber dem Führungsdraht 30a baulich leicht zu realisieren. Unter konzentrisch angeordnet soll verstanden werden, dass entweder der Hohlraum den Fluidraum umgibt oder der Fluidraum den Hohlraum umgibt. Es versteht sich, dass aufgrund der Flexibilität der Einführvorrichtung der Begriff "konzentrisch" nicht streng geometrisch zu verstehen ist. Figur 4 zeigt einen Schnitt durch ein Segment 10eines Führungskatheters 110 nach einem Ausführungsbeispiel der Erfindung mit nicht druckbeaufschlagtem Außenschlauch 114.

Durch den Führungskatheter 110 ist ein Innenschlauch 116 (auch Schaft genannt) geführt, der von einem Außenschlauch 114 (auch Schaft genannt) umgeben ist. Das Inneren des Innenschlauchs 116 bildet einen zentraler Bereich 34 des Führungskatheters 110. Zwischen Innenschlauch 116 und Außenschlauch 114 ist ein Fluidraum 115 gebildet, der den Innenschlauch 116 und damit den zentralen Bereich 34 umgibt. Durch den Fluidraum 115 kann ein Fluid geleitet werden, um den Führungskatheter 110 mit Druck zu beaufschlagen. Um den Außenschlauch 114 ist beabstandet eine Hülle 111b einer Schleuse angeordnet, wodurch ein Hohlraum 113 definiert ist. Der Außenschlauch 114 bildet eine Wand 50 zwischen Fluidraum 115 und Hohlraum 113. Die Wand weist im Kathetersegment 10 Wandöffnungen 52b auf.

Indem Kathetersegment 10 ist eine Membran 40b angeordnet, die den Außenschlauch 114 symmetrisch umschließt und dort angeordnete Mantelöffnungen 52b abdeckt. Denkbar ist auch eine unsymmetrische Anordnung der Membran 40b, etwa nur an bestimmten Bereichen des Umfangs des Außenschlauchs 114.

Die Membran 40a kann durch biegsame Materialien gebildet sein, oder auch durch andere, etwa halbfeste, Materialien gebildet sein, wie z.B. insbesondere Ethylen-Vinylacetat, Polyvinylchlorid, Olefinkopolymere, Olefinhomopolymere, Polyethylen-Typen (wie PE-HD bzw. HDPE, PE-LD bzw. LDPE, PE-LLD bzw. LLDPE, PE-HMW, PE-UHMW), Polyetheretherketon, Polyurethan, Silicone, Polyethylenterephthalat, Polyetherblockamid (PEBA), Acrylonitril-Polymere, Acrylonitril-Kopolymere, Acrylonitrilmischungen, Harze, insbesondere ionomere Harze. Andere geeignete Materialien können ebenfalls eingesetzt werden.

Figur 5 zeigt einen Schnitt durch das Kathetersegment 10 des Ausführungsbeispiels in Figur 4 mit druckbeaufschlagtem Führungskatheter 110. Durch den Fluidraum 115 gelangt ein Fluid in das Kathetersegment 10 und baut dort je nach Einsatzzweck einen Druck von einigen Bar bis 20 bar auf. Der vom Fluid aufgebaute Druck p drückt die Membran 40b von den Mantelöffnungen 52b nach außen, bis die Membran 40b an den die Hülle 111b stößt, der einen Anlagebereich 111 bildet, und diesen festklemmt. Die Lage und die Orientierung von Außenschlauch 114 und Schleuse sind nunmehr festgelegt, solange die Membran 40b auf den Anlagebereich 111 drückt. Wird der Druck p abgebaut, bewegt sich die Membran 40b wieder von ihrem Anlagebereich 111 in ihre Ausgangsposition zurück und gibt den Führungskatheter 110 frei. Durch die quasi "konzentrische" Anordnung von Hülle 111b, Hohlraum 113, Membran 40b, Außenschlauch 114, Fluidraum 115 und Innenschlauch 116 ist die Funktion der Fixierung des Führungskatheters 110 gegenüber der Schleuse baulich leicht zu realisieren.

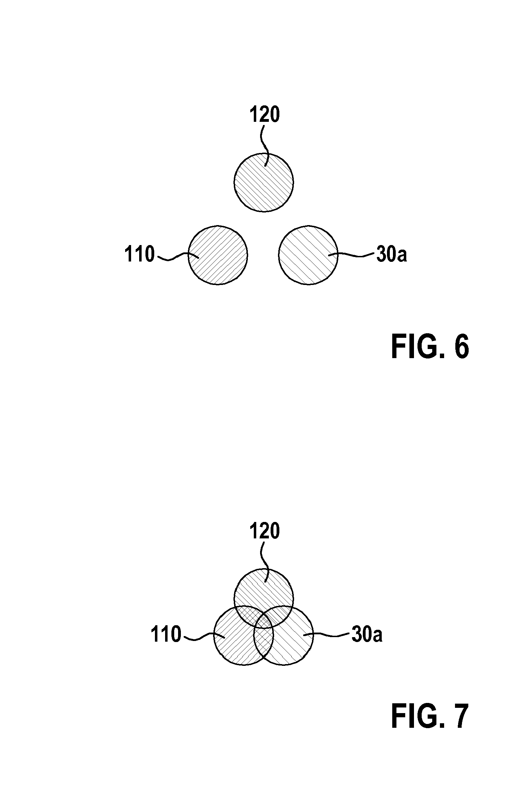

Die Figuren 6 und 7 veranschaulichen die Wirkung es beschriebenen Fixierungssystems der Einführvorrichtung. Figur 6 zeigt symbolisch einen Querschnitt durch einen Führungskatheter 110, einen Ballonkatheter 120 und einen Führungsdraht 30a einer Einführvorrichtung entsprechend Figur 1 mit einem entspannten Ballon (nicht dargestellt). Die genanten Komponenten sind in ihrer gegenseitigen Positionierung unbestimmt.

Figur 7 zeigt symbolisch einen Querschnitt durch einen Führungskatheter 110, einen Ballonkatheter 120 und einen Führungsdraht 30a einer Einführvorrichtung entsprechend Figur 1 mit einem druckbeaufschlagten Ballon (nicht dargestellt). Zwischen dem Ballon und dem fixiertem Führungsdraht und/oder Führungskatheter findet keine relative Bewegung statt. Nach der Ballon-Deflation kehren die elastischen Membranen zu ihrem ursprünglichen Zustand zurück.

### Bezugszeichen

- 100: Einführvorrichtung
- 102: proximales Ende
- 104: distales Ende

- 110: Führungskatheter
- 111: Hülle Schleuse Anlagebereich
- 112: Fixierungsposition Führungskatheter
- 113: Hohlraum
- 114: Außenschlauch
- 115: Fluidraum
- 116: Innenschlauch

- 120: Ballonkatheter
- 122: Fixierungsposition Führungsdraht
- 125: Fluidraum
- 126: Schlauch
- 128: Hülle

- 10: Kathetersegment
- 20: Kathetersegment

- 22: Ballon
- 24: Lumen
- 26: Ballonkatheter
- 30: Anlagebereich
- 30a: Führungsdraht
- 32: Hohlraum
- 34: zentraler Bereich
- 40a, 40b: Membran

- 50: Wand
- 52a, 52b: Öffnung

- p: Druck

## Patentansprüche

1. Einführvorrichtung (100), insbesondere Führungskatheter (110) und/oder Ballonkatheter (120), zum Einführen in einen tierischen und/oder menschlichen Körper, wobei wenigstens ein Kathetersegment (10, 20) vorgesehen ist, bei dem ein Hohlraum (113, 32) und ein Fluidraum (115, 125) um einen zentralen Bereich (34) angeordnet sind, wobei der Hohlraum (113, 32) und der Fluidraum (115, 125) durch eine Wand (50) getrennt sind und die Wand (50) wenigstens eine Wandöffnung (52a, 52b) aufweist, die durch eine elastische Membran (40a, 40b) abgedichtet ist, und wobei die elastische Membran (40a, 40b) ausgebildet ist, bei einer ausreichenden Druckdifferenz zwischen Fluidraum (115, 125) und Hohlraum (113, 32) zur Anlage an einem der wenigstens einen Wandöffnung (52a, 52b) gegenüberliegend angeordneten Anlagebereich (111, 30) im Hohlraum (113, 32) zu gelangen.

2. Einführvorrichtung nach Anspruch 1, wobei der Anlagebereich (111, 30) um denselben zentralen Bereich (34) wie Fluidraum (115, 125) und Hohlraum (113, 32) oder im zentralen Bereich (34) angeordnet ist.

3. Einführvorrichtung nach einem der vorhergehenden Ansprüche, wobei die wenigstens eine Wandöffnung (52a, 52b) durch Perforierungen gebildet ist.

4. Einführvorrichtung nach einem der vorhergehenden Ansprüche, wobei der Hohlraum (113) den Fluidraum (115) umgibt.

5. Einführvorrichtung nach Anspruch 4, wobei der Anlagebereich (111) einer Innenwand einer Hülle (111b) der Einführvorrichtung (100) zugeordnet ist.

6. Einführvorrichtung nach einem der Ansprüche, wobei der Anlagebereich (111) in einem Führungskatheter (110) angeordnet ist

7. Einführvorrichtung nach einem der Ansprüche 1 bis 3, wobei der Fluidraum (125) den Hohlraum (32) umgibt.

8. Einführvorrichtung nach Anspruch 7, wobei der Anlagebereich (30) einem Führungsdraht (30a) zugeordnet ist.

9. Einführvorrichtung nach einem der Ansprüche 1 bis 3, 7 oder 8, wobei der Anlagebereich (30) in einem Ballonkatheter (120) angeordnet ist.

10. Einführvorrichtung nach einem der vorhergehenden Ansprüche, wobei der Ballon (22) und/oder die Membran (40a, 40b) aus Nylon oder Polyamin gebildet ist

11. Einführvorrichtung nach einem der vorhergehenden Ansprüche, wobei der Ballon (22) und/oder die Membran (40a, 40b) aus einem Material der Gruppe gebildet ist: Ethylen-Vinylacetat, Polyvinylchlorid, Olefinkopolymere, Olefinhomopolymere, Polyethylen, Polyethylentypen, Polyurethan, Polyethylenterephthalat, AcrylonitrilPolymere, Acrylonitril-Kopolymere, Acrylonitrilmischungen, Harze, insbesondere ionomere Harze.

12. Einführvorrichtung nach wenigstens einem der vorhergehenden Ansprüche, wobei bei Druckbeaufschlagung der elastischen Membran (40a, 40b) Führungsdraht (30), Ballonkatheter (120) und Führungskatheter (110) in ihrer relativen Orientierung zueinander fixierbar sind.
